# FASCICULE DE BREVET EUROPEEN

(11) **EP 1 798 241 B1**
(45) Date de publication et mention de la délivrance du brevet: **19.01.2011**
(21) Numéro de dépôt: 06291910.5
(22) Date de dépôt: 12.12.2006
(51) Int. Cl.: C07K 16/06, C07K 16/44, C07K 14/765, C07C 237/48, G01N 33/94, A61K 47/48

(54) **Procédé de fabrication d'un complexe entre la doxycycline et l'albumine bovine sérique et son application à la fabrication d'un anticorps anti-doxycycline**
Methode zur Herstellung eines Doxycyclin-BSA Konjugates und dessen Anwendung zur Herstellung eines Anti-Doxycyclin Antikörpers
Method of manufacturing a conjugate of doxycycline and bovine serum albumin and its application for the manufacture of an anti-doxycycline antibody

(30) Priorité: 13.12.2005 FR 0512573
(43) Date de publication de la demande: 20.06.2007
(73) Titulaire: Etat Français représenté par le Délégué Général pour L'Armement, 94114 Arcueil Cedex (FR)
(72) Inventeur: Fusai Thierry, 13012 Marseille (FR)

(56) Documents cités:
- WO-A-2005/065382
- US-A- 3 042 716
- GOLDRING J P DEAN ET AL: "Raising antibodies in chickens against primaquine, pyrimethamine, dapsone, tetracycline, and doxycycline." IMMUNOLOGICAL INVESTIGATIONS. 2005, vol. 34, no. 1, février 2005 (2005-02), pages 101-114, XP008070280 ISSN: 0882-0139
- TEALE J D ET AL: "Radioimmunoassay of vinblastine and vincristine" BRITISH JOURNAL OF CLINICAL PHARMACOLOGY 1977, vol. 4, no. 2, 1977, pages 169-172, XP008070414
- FARAJ B A ET AL: "Development and application of a radioimmunoassay for tetracycline" JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 217, no. 1, 1 April 1981 (1981-04-01) , pages 10-14, XP008094231 ISSN: 0022-3565

## Description

L'invention concerne la recherche de la doxycycline dans l'urine ou le plasma humain ou animal.

Plus particulièrement, l'invention se rapporte à la préparation d'un haptène composé de doxycycline liée à l'albumine bovine sérique (BSA) pour permettre la production d'anticorps anti-doxycycline dans le but de détecter la doxycycline par une réaction immunologique.

La doxycycline est un antibiotique de la classe des tétracyclines de seconde génération et est utilisée, notamment, pour le traitement et la chimioprophylaxie antipaludique.

Il s'agit de la désoxy-6 hydroxy-5 tétracycline, de formule brute C₂₂H₂₄N₂O₈.

Depuis 2002, dans les armées, la prévention du paludisme résistant à la chloroquine est assurée en partie par l'utilisation de la doxycycline à raison de 100 mg par jour. Actuellement, la vérification de l'observance de cette chimioprophylaxie repose sur la mise en oeuvre de dosage dans les urines ou le plasma de la doxycycline par des techniques complexes nécessitant un laboratoire équipé de chromatographe liquide à haute performance (HPLC).

A l'évidence, ce procédé ne peut être utilisé sur le terrain. Or, pour permettre une gestion adaptée de la molécule, il semble indispensable que les médecins d'unité et l'encadrement disposent d'un test immédiat de suivi de la prise de doxycycline.

Il existe des tests immunologiques sous forme de kits pour la détection des tétracyclines, commercialisés notamment sous la marque RIDASCREEN par la société Charm Sciences Inc.

Ces tests sont basés sur la reconnaissance d'un antigène par un anticorps.

L'antigène provient du couplage de la tétracycline avec une protéine. Cependant, la révélation de l'anticorps se fait par des moyens optiques et nécessite l'emploi d'un spectrophotomètre. En outre, ces kits sont limités à la détection des tétracyclines en général à des fins alimentaires (dans le lait, le miel, la viande) et ne sont pas adaptés à une détection sensible chez l'homme ou l'animal.

Enfin, ces kits ne permettent de doser que certaines tétracyclines (tétracycline, chlortétracycline, minocycline, rolitétracycline, oxytétracycline) mais pas la doxycycline. Dans le Journal of Pharmacology and Experimental Therapeutics, 1980, 217, 1, 10-14, Bahjat A., Faraj et Farouk M. Ali décrivent un antisérum contre la tétracycline produit chez des lapins immunisés par un haptène formé de tétracycline conjugué à de la BSA. Cet antisérum est très sélectif, étant donné qu'il ne réagit secondairement qu'avec la chlortétracycline et que sa réactivité secondaire avec d'autres tétracyclines, dont la doxycycline, est négligeable (1%) . La détection est réalisée par marquage radioactif des molécules.

L'haptène est préparé par une réaction de Mannich, en mettant en contact la tétracycline avec la BSA, en présence de formaldéhyde.

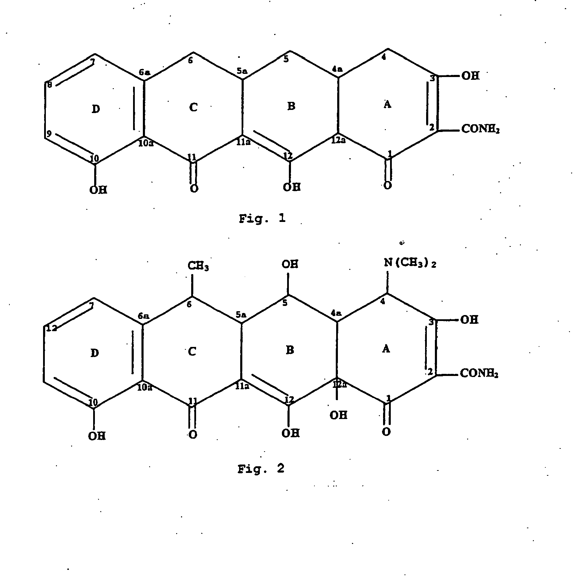

Il est connu d'appliquer la réaction de Mannich à des composés de la famille des tétracyclines, dont la structure générale est représentée sur la figure 1 ci-après, en les faisant réagir avec du formaldéhyde et des acides aminés. Le brevet américain 3 042 716 décrit des dérivés aminoalkylés obtenus par simple mise en contact en milieu aqueux et à température ambiante de ces composés avec du formaldéhyde et un acide aminé tel que la lysine. Les tétracyclines ont en commun un squelette de naphtacène constitué de 4 cycles benzéniques (A, B, C, D). L'alkylation a lieu sur le groupe amide situé en position 2 du cycle A. Ces nouveaux composés présentent une meilleure solubilité et une meilleure absorption gastro-intestinale.

Par contre, dans la publication de Bahjat et al., c'est l'atome d'hydrogène situé en position 9 du cycle D qui est impliqué dans la réaction.

Il est indiqué que la spécificité de l'antisérum résultant est influencée par divers facteurs, tels que la taille du pont entre l'haptène et la protéine porteuse, les degrés de liberté du site de liaison sur l'haptène. La réactivité de l'anticorps vis-à-vis des autres composés de la famille des tétracyclines par des substituants sur le noyau est directement proportionnelle à leur proximité avec celle de l'haptène. La plus grande affinité de l'anticorps pour l'haptène est pour la zone la plus éloignée du site d'attachement de la protéine. L'introduction d'un groupe hydroxyle en position 5 du noyau B, comme dans la doxycycline (désoxy-6 hydroxy-5 tétracycline), dont la formule développée est représentée sur la figure 2 ci-après, réduit considérablement l'affinité de ces composés pour l'anticorps. Le but de la présente invention est la mise au point d'un procédé d'identification de la doxycycline dans les urines ou le plasma de l'homme ou de l'animal qui soit utilisable sur le terrain sans nécessiter un appareillage complexe, et qui soit rapide, spécifique, sensible et peu onéreux.

Pour ce faire, on a eu l'idée de créer un composé antigénique capable d'induire la production d'anticorps anti-doxycycline. L'invention a pour objet un procédé de fabrication d'un haptène entre un dérivé de la tétracycline et l'albumine bovine sérique par une réaction de Mannich, **caractérisé en ce que** le dérivé de la tétracycline est la doxycycline et que le procédé comprend les étapes suivantes :
a) dissolution de la doxycycline et de l'albumine bovine sérique en présence de formaldéhyde à un pH compris entre 4 et 5,
b) incubation du mélange de l'étape précédente à une température comprise entre 25°C et 50°C pendant une durée égale ou supérieure à 12 heures,
c) purification et isolement de la doxycycline couplée à la BSA par passage sur une colonne de dessalage du mélange réactionnel de l'étape précédente.

Selon un mode de réalisation optimisé, l'incubation est effectuée à une température de 40°C pendant 12 heures. L'étape a) est réalisée à un pH de 4,7.

L'invention a également pour objet l'utilisation de l'haptène fabriqué selon l'invention à l'identification de la doxycycline dans l'urine de l'homme ou de l'animal.

Comme indiqué dans l'article de Bahjat et al . précédemment cité, dans les mêmes conditions expérimentales, l'identification, par ce procédé, de la doxycycline est 100 fois moins sensible par rapport à la sensibilité de la tétracycline.

Il a donc fallu mettre au point des conditions opératoires particulières, telles que l'acidité du milieu et la température, pour obtenir un rendement de couplage entre la doxycycline et la BSA acceptable. Le rendement de couplage a été estimé par le dosage de la doxycycline libre avant et après couplage.

La doxycycline possède un hydrogène réactif en position 9 du cycle D. Ce dernier peut réagir avec un acide aminé en présence de formaldéhyde selon une réaction de Mannich. La réaction a lieu en particulier avec les résidus lysine présents en grand nombre (9% des acides aminés)de la BSA. On obtient ainsi un polymère de doxycycline-BSA.

Ce polymère est une structure antigénique capable d'induire la production d'anticorps spécifiques anti-doxycycline. L'invention sera mieux comprise à l'aide des exemples de réalisation ci-après.

### Exemple de fabrication d'un haptène doxycycline-BSA selon l'invention :

On utilise 2mg de BSA cationique lyophilisée riche en lysine connue sous la dénomination commerciale SuperCarrier Immune Modulator fabriqué par la Sté PIERCE.

On dissout ce lyophilisat dans 200µl d'eau ultra-pure MilliQ® fabriquée par la Sté Millipore. On obtient une solution de BSA à 10mg/l contenant 0,1 M MES[Acide (N-morpholino)-2 éthanesulfonique], 0,15 M NaCl, des agents de stabilisation, à pH 4,7.

On dissout 5mg d'hydrochlorure de doxycycline fabriqué par la Sté SIGMA dans 200µl d'une solution de tampon de liaison comprenant 100 M MES, 0,15 M NaCl, de pH 4,7.

On mélange les 200µl de BSA avec les 200µl de doxycycline.

On additionne au mélange 50µl d'une solution contenant 37% de formaldéhyde fabriquée par la Sté SIGMA.

On incube le mélange réactionnel à 40°C pendant 12 heures.

On utilise une colonne de dessalage D-Salt® Dextran Plastic Desalting Column fabriquée par la Sté PIERCE et on l'équilibre avec 5ml d'une solution de tampon de purification contenant 6mM PBS, 150mM NaCl, de pH 7,2.

On dépose sur la colonne la totalité du mélange réactionnel.

On récupère la totalité de l'échantillon dans un 1^{er} tube (fraction non tamisée).

On dépose successivement, toujours au centre de la colonne, 17 fois 1ml de tampon de purification.

On recueille à chaque fois les fractions qui sortent de la colonne dans les tubes numérotés de 2 à 18.

On dose la protéine pour identifier les fractions d'intérêt. L'estimation du rendement de ce couplage a été réalisée par chromatographie à haute pression (HPLC).

Le calcul du rendement est réalisé par le dosage de la doxycycline libre.

Deux séries de couplages ont été réalisées et le dosage de la doxycycline libre dans chaque échantillon a été comparé par rapport à un blanc correspondant à la purification de la doxycycline en l'absence de BSA.

Les résultats figurent dans le tableau ci-dessous.

| **Fraction** | **Série 1 (µg/ml)** | **Série 2 (µg/ml)** | **Blanc sans BSA (µg/ml)** |
|---|---|---|---|
| 1 | 0,00 | 0,00 | 0 |
| 2 | 0,00 | 0,00 | 0 |
| 3 | 0,00 | 1, 18 | 0 |
| 4 | 1,36 | 2,24 | 2,23 |
| 5 | 3,63 | 4,64 | 145,579 |
| 6 | 25,14 | 3,72 | 451,355 |
| 7 | 41, 34 | 30,56 | 626,279 |
| 8 | 15,86 | 36,84 | 1170,285 |
| 9 | 23,82 | 10,80 | 1045,282 |
| 10 | 9,85 | 28,24 | 684,039 |
| 11 | 18,21 | 9,13 | 277, 967 |
| 12 | 12,51 | 50,16 | 111, 416 |
| 13 | 55,82 | 66,24 | 34,145 |
| 14 | 41,07 | 51,88 | 16,174 |
| 15 | 39,44 | 44,56 | 7,183 |
| 16 | 38,88 | 28,88 | 5,044 |
| 17 | 19,85 | 19,20 | 2,924 |
| 18 | 23,93 | 13,14 | 1,875 |
| Total | 370,73 | 401,41 | 4581,777 |

Le rendement varie entre 81% et 87,5%, comme indiqué dans le tableau ci-après.

| Série | Quantité de doxycycline libre (µl) | Rendement (%) |
|---|---|---|
| 1 | 370,73(+/-10%) | 81,0 |
| 2 | 401,41(+/-10%) | 87, 5 |
| Blanc sans BSA | 4580 | |

On a ensuite cherché à valider l'hypothèse d'une réponse anti-doxycycline en immunisant des souris, selon le protocole décrit ci-dessous.

### Essai d'immunisation :

On a procédé à un essai d'immunisation sur un lot de souris.

Les souris ont reçu chacune 4 injections intra-péritonéales, à un mois d'intervalle, de 500µl d'une suspension contenant 100µg/ml de BSA-doxycycline et de l'alun à 50% en volume.

L'analyse de la réponse après la dernière injection a été faite à partir d'un test ELISA de la manière suivante.

Une plaque de micro-titration a été sensibilisée avec une solution contenant 10µ/ml sous 50µl/puits de BSA-doxycycline en présence de tampon carbonate/bicarbonate durant 12 heures à 4°C.

Les sérums des souris immunisées ont été incubés pendant 2 heures à 37°C sous une dilution de 1/500, 1/1000 ou 1/1500 seuls ou en présence d'une solution de doxycycline de concentration décroissante à partir de 40µg/ml, respectivement, 40, 20, 10, 5µg/ml.

Enfin, la révélation a été faite par un anti-corps anti-souris conjugué à la peroxydase.

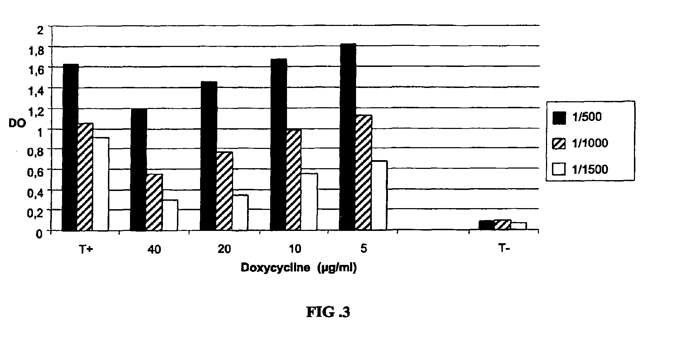

Les résultats sont illustrés sur la figure 3 ci-après où les T⁺ représentent les témoins positifs sans doxycycline libre et les T⁻ représentent les témoins négatifs, c'est-à-dire les puits non sensibilisés.

La chute de la réponse en présence de doxycycline libre (évaluée en densité optique DO) montre qu'au décours des immunisations, on a obtenu une réponse anti-doxycycline spécifique chez la souris, ce qui valide la construction et l'hypothèse.

### Exemple de préparation de solutions pour le dosage urinaire de la doxycycline chez l'homme :

On rappelle que la doxycycline est utilisée dans la prophylaxie du paludisme résistant à la chloroquine à la posologie de 100mg/jour.

Pour une prise de 200mg, le pic sérique est de 3µg/ml.

La concentration résiduelle à 24 heures est de 1µg/ml.

La demi-vie sérique est de 16 à 24 heures.

La liaison aux protéines est supérieure à 85%.

La concentration urinaire est 10 fois plus élevée que la concentration dans le sang au même instant.

On prépare des solutions de doxycycline de dilution croissante contenant respectivement 100, 85, 75, 60, 50, 25, 20, 10µg/ml, à partir d'une solution mère obtenue en dissolvant 10,8mg de doxycycline HCl dans 10ml d'eau ultra-pure MilliQ® fabriquée par la Sté Millipore.

Les solutions filles sont aliquotées par volume de 2ml et mises à -80°C.

La préparation des échantillons d'urine se fait par centrifugation des urines à 4000 tours/mn pendant 5 minutes, puis dilution au 1/20 (50µl d'échantillon dans 1ml d'eau ultra-pure).

On injecte 80µl des dilutions au 1/20 préparées précédemment dans un chromatographe HPLC.

## Revendications

1. Procédé de: fabrication d'un complexe d'un haptène et d'une molécule poreuse entre un dérivé de la tétracycline et l'albumine bovine sérique par une réaction de Mannich, **caractérisé en ce que** le dérivé de la tétracycline est la doxycycline et que le procédé comprend les étapes suivantes :
a) dissolution de la doxycycline et de l'albumine bovine sérique en présence de formaldéhyde à un pH compris entre 4 et 5,
b) incubation du mélange de l'étape précédente à une température comprise entre 25°C et 50°C pendant une durée égale ou supérieure à 12 heures,
c) purification et isolement de la doxycycline couplée à la BSA par passage sur une colonne de dessalage du mélange réactionnel de l'étape précédente.

2. Procédé selon la revendication 1, **caractérisé en ce que** l'incubation est effectuée à une température de 40°C pendant 12 heures.

3. procédé selon la revendication 2, **caractérisé en ce que** l'étape a) est réalisée à un pH de 4,7.

4. Utilisation d'un complexe d'un haptène et d'une molécule porteuse fabriqué selon les revendications 1 à 3 à la production d'un anticorps anti-doxycycline permettant, par une réaction immunologique, l'identification de la doxycycline dans l'urine ou le plasma de l'homme ou de l'animal.

## Claims

1. Process for the manufacture of a complex of a hapten and a carrier molecule between a tetracycline derivative and bovine serum albumin by a Mannich reaction, **characterized in that** the tetracycline derivative is doxycycline and that the process comprises the following steps:
a) dissolution of the doxycycline and the bovine serum albumin in the presence of formaldehyde at a pH between 4 and 5,
b) incubation of the mixture from the previous step at a temperature between 25°C and 50°C for a period greater than or equal to 12 hours, and
c) purification and isolation of the doxycycline coupled to the BSA by passing the reaction mixture from the previous step over a desalting column.

2. Process according to Claim 1, **characterized in that** the incubation is effected at a temperature of 40°C for 12 hours.

3. Process according to Claim 2, **characterized in that** step a) is carried out at a pH of 4.7.

4. Use of a complex of a hapten and a carrier molecule manufactured according to Claims 1 to 3 for the production of an anti-doxycycline antibody that makes it possible, by an immunological reaction, to identify doxycycline in human or animal urine or plasma.

## Patentansprüche

1. Verfahren zur Herstellung einer Komplexverbindung eines Haptens und eines Trägermoleküls zwischen einem Tetracyklinderivat und dem Rinderalbuminserum durch eine Mannich-Reaktion, **dadurch gekennzeichnet, dass** das Tetrazyklinderivat Doxycyclin ist und dass das Verfahren folgende Schritte umfasst:
a) Auflösung des Doxycyclin und des Rinderalbuminserums in Gegenwart von Formaldehyd bei einem pH-Wert zwischen 4 und 5,
b) Inkubation des Gemischs aus dem vorhergehenden Schritt bei einer Temperatur zwischen 25°C und 50°C während einer Dauer von mindestens 12 Stunden,
c) Reinigung und Isolation des mit dem BSA gekoppelten Doxycyclins, dadurch, dass man das Reaktionsgemisch aus dem vorhergehenden Schritt durch eine Entsalzungssäule laufen lässt.

2. Verfahren nach Anspruch 1, **dadurch gekennzeichnet, dass** die Inkubation bei einer Temperatur von 40°C während 12 Stunden durchgeführt wird.

3. Verfahren nach Anspruch 2, **dadurch gekennzeichnet, dass** der Schritt a) bei einem pH-Wert von 4,7 durchgeführt wird.

4. Verwendung einer nach den Ansprüchen 1 bis 3 hergestellten Komplexverbindung eines Haptens und eines Trägermoleküls bei der Erzeugung eines Anti-Doxycyclin-Antikörpers, der es ermöglicht, durch eine immunologische Reaktion Doxycyclin im Urin oder im Plasma des Menschen oder des Tieres zu identifizieren.
